# EUROPEAN PATENT APPLICATION

(11) **EP 1 695 735 A1**
(43) Date of publication of application: **30.08.2006**
(21) Application number: 04819894.9
(22) Date of filing: 02.12.2004
(51) Int. Cl.: A61N 1/36

(54) **NONPHARMACOLOGICAL GENERATIVE FUNCTION CONTROL METHOD AND ITS APPARATUS**

(30) Priority: 02.12.2003 JP 2003402739
(71) Applicant: Hakuju Institute for Health Science Co., Ltd, Tokyo 151-0063 (JP)
(72) Inventor: SUZUKI, Hiroshi, c/o Obihiro University, Obihiro, Hokkaido 080-8555 (JP); HORI, Takuya, Hakuju Inst for Health Sci. Co, Ltd, Tokyo 151-0063 (JP); HARAKAWA, S., Hakuju Inst. for Health Sci Co, Ltd, Tokyo 151-0063 (JP)
(74) Representative: Senior, Janet
(86) International application number: PCT/JP2004/017942
(87) International publication number: WO 2005/053790

(57) **Abstract**

The method treats or improves generative function of male animals nonpharmacologically or the method controls sexual behavior of male animals. Electrodes are exposed to said male animals in a non-contact state and a low frequency AC electric field is exposed to a whole or a part of said male animals. Said electric field is exposed for a prescribed time period of a day and the exposure is repeated every day.

## Description

The description of this application claims benefit of priority based on Japanese Patent Application No.2003-402739, the entire same contents of which are incorporated by reference herein.

### BACKGROUND OF THE INVENTION

### Field of the Invention

The present invention relates to the nonpharmacological generative function control method of male animals and its apparatus.

### Description of the Prior Art

Statistically, it is said that couples with no children for more than two years in their conjugal life have high possibility of infertility. It is estimated that the number of couples suffering from infertility is 1, 800, 000 in Japan and the number tends to increase. Among them, it is said that the number derived from male infertility is about 700,000 (about 40%). Sexual dysfunction includes erectile dysfunction, ejaculation dysfunction, or decreased libido and lack of orgasm, and the like and among them, the erectile dysfunction is one of the main. Causes of the erectile dysfunction are classified into psycogenic erectile dysfunction caused by stress and the like and organic erectile dysfunction caused by diabetes, by the operation within pelvis, and the like.

Among patients, the number of those who suffer from erectile dysfunction is estimated to be about 9, 800, 000 in Japan but the ratio of those who actually undergo treatment at medical institutions is only about 3 %, which shows that men hesitate to consult a doctor for the dysfunction. For information, a therapeutic medicament described in Patent Document 1 is known for treating human erectile dysfunction.
Patent Document 1: Japanese Patent Laid-Open Publication No. Hei 11-263728.

Further, not limited to human beings, in breeding domestic animals such as cattle, horses including race horses, and the like, decrease in copulation rate is a serious problem that horse owners and cattle farmers are facing and they have been longing for methods to improve breeding performance.

As mentioned above, in treating erectile dysfunction, method of administering a therapeutic medicament is employed. However there still remains the problem of side effect caused by therapeutic medicaments. Therefore, other methods for improving or treating sexual dysfunction have been longed for. In addition, methods for improving breeding ability by controlling (promoting) sexual behavior of animals have also been longed for

The object of the present invention is to provide a method for controlling sexual dysfunction by a nonpharmacological method. Further, the object of the present invention is to provide a method for treating or improving sexual dysfunction by a nonpharmacological method and its apparatus.

### SUMMARY OF THE INVENTION

In order to solve the above mentioned problems, as a result of intensive studies, it has been found that nonpharmacological generative function control method of male animals, wherein electric field is exposed to whole or a part of said male animals with electrodes in a non-contact state with said male animals can be provided.

In the present invention, "non-contact" in the present invention means that there is no contact for flowing electric current between two or more electrodes. Therefore, the "non-contact" in the present invention includes the case where a male animal contacts with one electrode but does not contact with other electrode couple or where the male animal contacts with a plurality of identical electrodes but does not contact with other electrode couple in addition to the case where the male animal does not contact with both electrodes. The "non-contact" state in the context that the male animal does not contact with both electrodes is desirable.

The treating or improving method of the present invention is a method for treating or improving sexual dysfunction by exposing a male animal to electric field and controlling generative function. Although the relation between electric field and generative function is not found out in detail, it is assumed that NO concentration in blood considered to be deeply involved with generative function increases. Relaxation effect in the blood vessel that NO has has already been understood and it is considered that penile erection occurs since the relaxation by NO presses peripheral vein gapping urethra corpus cevernosum trabecular smooth muscle, and the arteriolar expansion in corpus cevernosum increases the blood flow volume.

It is also considered that secretion of testosterone indispensable for expressing and maintaining male secondary sexual characteristic, cortisol known as stress index, ACTH, and hormones considered to be involved with promoting copulation behavior is promoted thereby treating or improving sexual dysfunction.

Since the present invention is not a method of flowing electric current but a method of exposing electric field, an electrode and a male animal are placed in non-contact.

For information, said electric field is preferably a low frequency AC electric field.

In addition, the electric field is desirably exposed to generative organs of said male animal.

Therefore, considering penetration of the electric field inside of a body of the male animal, it is desirable to have AC current having low frequency, and for the purpose of controlling generative function to treat and improve erectile dysfunction, it is preferable that the electric field is at least exposable to generative organs.

Regarding exposure, it is preferable that said electric field is exposed only for a certain period of time in a day repeating this every day. By exposing said electric field only for a certain period of time in a day and by repeating this every day, generative function can be controlled and treatment or improvement in sexual dysfunction can be obtained.

Although generative function can be treated or improved by controlling the generative function, obstacles to this are concerned which include erectile dysfunction, ejaculation dysfunction, decrease in libido or lack in orgasm.

By the electric field exposure, it is considered that increase of NO concentration in the blood and the promotion in hormone secretion are obtained, which acts as the effective method for treating erectile dysfunction, ejaculation dysfunction, decrease in libido or lack in orgasm.
Male animals are animals which include human beings and the species
thereof are not specifically limited, however, the method is particularly effective for domestic animals such as horses including race horses, cattle, pigs, and the like as well as for human beings.

In addition, regarding other embodiments of the present invention, a method for controlling sexual behavior of animals can be provided using controlling method of generative function set forth in any of the above mentioned embodiments.

For example, the method relates to a controlling method of sexual behavior by exposing electric field to said whole or a part of said animals. Further, by exposing electric field to both male and female animals at the same time to control the sexual behavior of the male animals can also be employed.

By these methods, sexual behavior of animals can be controlled (promoted). Since exposure to electric field is considered to promote hormone secretion, by exposing electric field to both male and female animals, sexual behavior of such animals can be controlled. For example, by exposing electric field to domestic animals such as horses including race horses, cattle, pigs, and the like, breeding ability can be improved.

In addition, another embodiment of the present invention is a generative function control apparatus for treating or improving sexual dysfunction of male animals, wherein said generative function control apparatus is provided with a pair of electrodes with electric field exposable to a whole or a part of said male animals and a power supply which applies voltage to said pair of electrodes.

By this apparatus, generative function of male animals can be controlled thereby capable of treating or improving sexual dysfunction.

Further, as the other embodiment of the present invention, it is preferable that said apparatus is provided with a pair of electrodes arranged with a prescribed interval so that said male animals or a part thereof might be placed and a power supply for generating electric field between said pair of electrodes by applying prescribed voltage to said pair of electrodes.

This apparatus is the apparatus which applies voltage to a pair of electrodes thereby exposing male animals to electric field generated between said electrodes. As mentioned above, it is considered that there is relationship between electric field and generative function and therefore, it is effective in treating or improving sexual dysfunction of male animals.

It is further preferable that said electric field is a low frequency AC electric field.

Further, it is preferable that this apparatus is provided with a power supply controller in which at least applied voltage, frequency, and exposure time can be controlled.

Moreover, it is preferable that the apparatus can expose at least generative organs of said male animals to said electric field.

Considering penetration of the electric field inside of a body of the male animal, it is desirable to have AC current having low frequency and for the purpose of treating and improving erectile dysfunction, it is preferable that the electric field is at least exposable to generative organs.
In addition, depending on size or weight of male animals to which electric field is exposed, it is preferable that at least one can be controlled among applied voltage (exposed electric field intensity), frequency, and exposure time.

Said sexual dysfunction includes erectile dysfunction, ejaculation dysfunction, decrease in libido or lack in orgasm.

As mentioned above, since it is considered that NO concentration in blood increases or hormone secretion is promoted by the electric field exposure, the apparatus is effective in treating or improving erectile dysfunction, ejaculation dysfunction, decrease in libido, or lack in orgasm.

In addition, in this apparatus, it is desirable that said pair of electrodes is arranged in a non-contact state with said male animals.

Since it is an apparatus for exposing electric field, there is no need to apply current to male animals, it can make electrodes and male animals in a non-contact state. Therefore, there is no need to constrain said male animals by the electrodes.

Further, this apparatus is applicable to human beings and mammals as said male animals, and in particular, applicable to horses, cattle, pigs, and mice as mammals.

In other words, male animals include animals including human beings and although the species thereof are not specifically limited, other than human beings, it is particularly effective for domestic animals (mammals) such as horses including race horses, cattle, and pigs.

It is preferable that this apparatus is provided with a placing table on which whole or a part of said male animals can be placed in a stable state.

By adding the placing table placed in a stable state, the movement of male animals to be tested can be suppressed to the minimum without constraining them. As a result, electrodes which sandwich said male animals therebetween can be suppressed to the requisite minimum.

It is preferable that said male animals are human beings and that said placing table is a chair provided with a back rest portion, a seat plate portion, and a foot rest portion on which said human beings can sit.

Further in this apparatus, it is preferable that one of said pair of electrodes is arranged upward of a head portion of said human beings by an electrode supporting portion which extends from the back rest portion of said chair and that the other of said pair of electrodes is arranged on said foot rest portion.

When male animals are human beings and when this apparatus is provided with a chair on which human beings can sit, sexual dysfunction can be treated or improved in a seated, relaxed state. In particular, by arranging electrodes upward of a head portion or on a foot rest portion, human beings can be exposed to electric field from a head portion to a foot portion of human beings, thereby providing an apparatus which treats or improve sexual dysfunction effectively.

These apparatuses are the apparatuses which control (promote) sexual behavior of animals. Since it is considered that exposure by electric field promotes secretion of hormone, it is the apparatus capable of controlling sexual behavior of said animals (both male and female) by exposing electric field. For example, by exposing electric field to domestic animals such as horses including race horses, cattle, pigs, and the like, breeding capability can be improved.

### BRIEF DESCRIPTION OF THE DRAWINGS

Fig.1 is a schematic view showing one example of electric field treating and improving apparatus of the present invention.
Fig.2 is a schematic view showing the electric field treating and improving apparatus of the present invention used in Example 1.
Fig. 3 is a graph showing a change in body weight of C57BL/6J mice with the number of days in exposure groups and control groups respectively.
Fig.4 is a schematic view showing the electric field treating and improving apparatus of the present invention used in Example 2.
Fig.5 is a schematic view showing the electric field exposure cage of the present invention used in Example 2.
Fig.6 is a graph showing the mating rate with the number of days for electric field exposure in Example 2.

### DETAILED DESCRIPTION OF THE PREFERRED EMBODIMENT

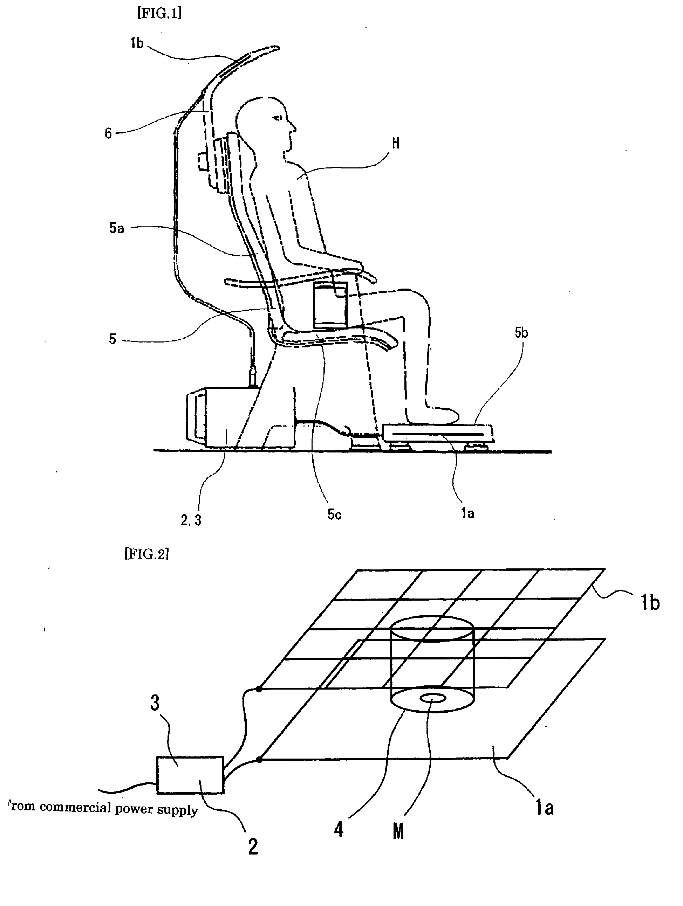

Hereinafter, referring to the drawings, embodiments of the present invention are explained. Fig.1 is a schematic view showing one example of electric field treating and improving apparatus of the present invention. The apparatus shown in the figure is the apparatus exposing a human being H (male) sitting on a chair 5 (a placing table) provided with a back rest portion 5a, a foot rest portion 5b, and a seat plate portion 5c to electric field. Inside of the foot rest portion 5b, one of a pair of electrodes 1a is arranged. The other of a pair of electrodes 1 b is attached to an electrode supporting portion 6 extending from the back rest portion 5a. In other words, since there is a gap between a head portion of a human being H and the electrode 1 b, they do not contact with each other, and since the electrode 1 a is inside of the foot rest portion 5b, it does not contact with a foot portion of the human being H. Therefore, a pair of electrodes 1 is arranged in a non-contact state with the human being H.

By applying voltage from an AC power supply 2 to a pair of electrodes 1a and 1b, electric field is generated between a pair of electrodes 1. By such arrangement of the pair of electrodes 1, electric field can be exposed not only to generative organs but also to a head portion through a foot portion of the human being H. Further, an AC power supply 2 can control frequency, applied voltage, exposure time, and the like by a controller 3, and depending on body height and body weight of the human being H, these numerical values can be controlled. For information, considering penetration of the electric field inside of the body of the human being H, it is desirable to have AC current having low frequency.

As mentioned above, although the relationship between electric field and generative function is not understood in detail, by electric field exposure, it is considered that NO concentration in the blood of a human being H increases or/and secretion of hormone assumingly involved with generative function of copulation behavior is/are promoted thereby treating or improving sexual dysfunction of the human being H. In addition, by exposing electric field for a prescribed time period in a day and repeating it everyday, the hormone is considered to be promoted thereby showing a treatment (improvement) effect.

Although methods and apparatuses for human beings have been explained, treatment and improvement for other male animals such as horses including race horses, cattle, pigs, and the like are also applicable by preparing an appropriately sized pair of electrodes 1 and by arranging them with appropriate intervals provided therebeween. Also since it is considered that secretion of hormone assumingly involved with promoting mating behavior is promoted by electric field exposure, sexual behavior of animals (both male and female) can be controlled (promoted) . Hereinafter, explanation goes in Examples regarding the methods and apparatuses of the present invention.

### EXAMPLE

### (Example 1)

In order to confirm the effectiveness of treatment of sexual dysfunction and improvement methods by the electric field exposure of the present invention, experiments were conducted using mice. 60 of C57BL/6J male mice (5 weeks age, body weight: 17 to 20 g) and 60 of female mice (7 weeks age, body weight: 18 to 21 g) of the same strain were obtained from CLEA Japan Inc. The mice were taken out for experiments after acclimated to the environment in a SPF (Specific Pathogen Free) room (temperature: 25±5°C, moisture: 50±5%, light period: 07:00 to 19:00).

In addition, prior to this, inventors conducted experiments on copulation for two couples, i.e, ICR male mice and C57BL/6J female mice, and C57BL/6J male mice and C57BL/6J female mice. According to table 1 which shows experimental results, compared with ICR male mice, C57BL/6J male mice had lower mating rate. Therefore, when the copulation rate of said mice improves, it can be said that treating and improving methods for sexual dysfunction of the present invention are effective.

**(Table 1)**

| Strain | | Mating rate (%) |
|---|---|---|
| Male | Female | |
| ICR | C57BL/6J | 100 |
| C57BL/6J | C57BL/6J | 51 |

| | | |
|---|---|---|
| Mating was evaluated by observing establishment of copulation by letting males live together with superovulated females overnight and detecting the following day. | | |

Time period for the acclimation of male mice (C57BL/6J) and female mice (C57BL/6J) was set for 19 days (8 weeks age) and 29 days (11 weeks age), respectively.

For electric field exposure, electric field exposure apparatus for mice (an apparatus for electric field treatment and improvement) shown in Fig.2 was used. Voltage is applied to a pair of electrodes 1a and 1b from an AC power supply 2. An electrode 1a is a plate made of stainless steel (SUS) and an electrode 1b is meshed for ventilation and the size of both electrodes is 60cm squares, respectively. An acrylic cylindrical spacer 4 (diameter 20 cm, height 20 cm) is inserted in the substantial center between electrodes 1a and 1b and in the spacer 4, male mice Mare placed. Therefore, uniformelectric field can be exposed to the mice with electrodes in a non-contact state.

The experiment was conducted under the exposure conditions of frequency of 50Hz, electric field intensity of 45 kV/m, and exposure time period of 30 minutes a day, setting the time span 10:30 to 14:00, which are controlled by a controller 3. The comparative groups were handled under the same conditions of the exposure groups except that the electric field exposure was set to be 0kV/m.

After the electric field exposure for 11 days, male mice of the electric field exposure group and those of a comparative group were let to live together with female mice of the same strain from the 11 th day at 17:00 to 9:00 of the following day (16 hours). Regarding female mice, for superovulation treatment, 2 days before the copulation, intraperitoneal injections of equine chorionic gonadotropin (5 IU) and right before the copulation, intraperitoneal injections of human chorionic gonadotrophin (5 IU) were applied to them, respectively. From 9: 00 of the following day of the mating, establishment of copulation was determined by detection of a vaginal plug, and from 18:00 of the same day, ovarian ducts were obtained from female mice by dislocating cervical vertebra thereof, thereby collecting eggs by circulation fluids of the ovarian ducts. The presence of pronucleus cells (presence of fertilization) was observed. These experiments were conducted three times repeatedly using 10 mice of the exposure group and 10 mice of the comparative group (Experiments 1 to 3).

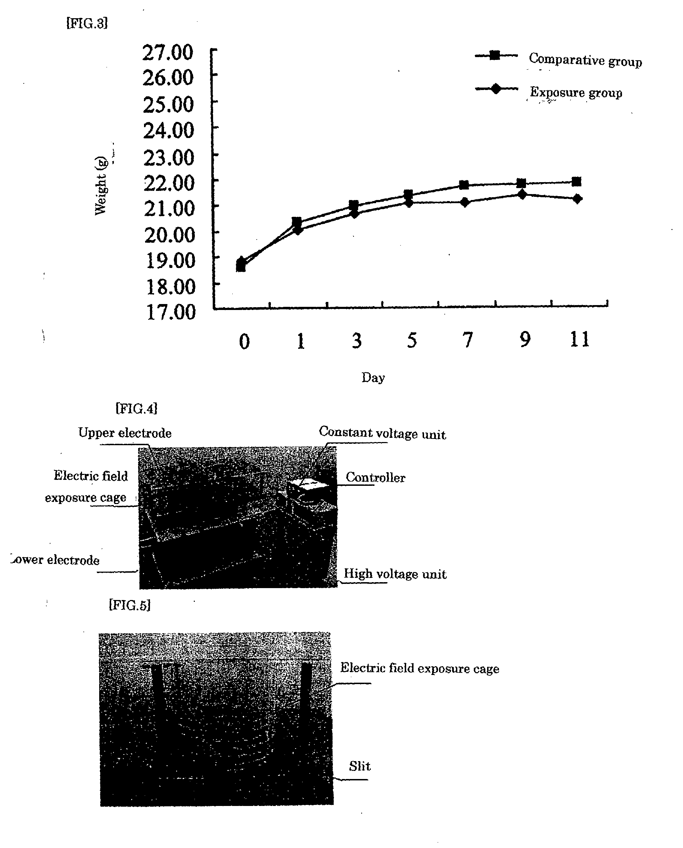

According to the observation by the naked eye, no difference was observed between experimental sections including behavioral abnormality during, before, and after the electric field exposure. Fig.3 shows the effect of electric field exposure on the change of body weight of C57BL/6Jmice in exposure groups and comparative groups, respectively. The change in the body weight during the period of electric field exposure for 11 days was shown by the average values. The number of animals n is 30.

According to Fig.3, almost no difference was observed in the change of body weight between the groups and since both groups show increase in the body weight, it is acknowledged that the electric field exposure used in the present experiments does not affect the growth of individual mouse.

Establishment rate of mating (comprehensive evaluation) is shown in table 2. The result of establishment of mating in experiments 1 to 3 is shown in table 3. The rate of female mice observed to have established mating on the following day of the establishment of the mating was 90 % in the electric field exposure groups, while 63 % in the comparative groups (student's t test, P<0.05) .

In tables 2 and 3, the number of animals is shown in electric field groups and in comparative groups in both case that copulation was established or not. And numbers shown in parentheses shows the rate (%) of the number of said animals.

**Table 2 Comprehensive evaluation**

| Effect of the electric field exposure on the copulation rates in C57BL/6J mice -1 | | |
|---|---|---|
| Number of mice with vaginal plugs detected (rate) | | |
| Groups | Electric field exposure groups | Comparative groups |
| Mating established | 27 (90%) | 19 (63%) |
| Mating not established | 3 (10%) | 11 (37%) |

**Table 3 Evaluation of each experiment**

| Effect of the electric field exposure on the copulation rates in C57BL/6J mice -1 | | | | | | |
|---|---|---|---|---|---|---|
| Number of mice with vaginal plugs detected (rate) | | | | | | |
| | Experiment 1 | | Experiment 2 | | Experiment 3 | |
| Groups | Electric field exposure groups | Comparative Groups | Electric field exposure groups | Comparative Groups | Electric field exposure groups | Comparative Groups |
| Mating established | 8 (80%) | 5 (50%) | 10 (100%) | 7 (70%) | 9 (90%) | 7 (70%) |
| Mating not established | 2 (20%) | 5 (50%) | 0 (0%) | 3 (30%) | 1 (10%) | 3 (30%) |

The rate of fertilized eggs (comprehensive evaluation) is shown in table 4. Table 5 shows the rate of fertilized eggs in each experiment of experiments 1 to 3. From table 4, the rate of female mice whose fertilized eggs were obtained in the following day of mating was 86.7 % in electric field exposure groups, while the rate is 56.7 % in comparative groups (student's t test, P<0.05) .

**Table 4 Comprehensive evaluation**

| Effect of the electric field exposure on the copulation rates in C57BL/6J mice -2 | | |
|---|---|---|
| Number of mice with fertilization acknowledged (rate) | | |
| Groups | Electric field exposure groups | Comparative groups |
| Fertilized eggs | 26 (86.7%) | 17(56.7%) |
| No fertilized eggs | 4 (13.3%) | 13(43.3%) |

**Table 5 Evaluation of each experiment**

| Effect of the electric field exposure on the copulation rates in C57BL/6J mice -2 | | | | | | |
|---|---|---|---|---|---|---|
| Number of mice with fertilization acknowledged (rate) | | | | | | |
| | Experiment 1 | | Experiment 2 | | Experiment 3 | |
| Groups | Electric field exposure groups | Comparative Groups | Electric field exposure groups | Comparative Groups | Electric field exposure groups | Comparative Groups |

| | | | | | | |
|---|---|---|---|---|---|---|
| Fertilization eggs | 8 (80%) | 5 (50%) | 10 (100%) | 6 (60%) | 8 (80%) | 6 (60%) |
| No fertilization eggs | 2 (20%) | 5 (50%) | 0 (0%) | 4 (40%) | 2 (20%) | 4 (40%) |

### (Example 2)

C57BL/6J male mice (7 weeks age), female mice (7 weeks age) of the same strain, and ICR male mice (7 weeks age) were obtained from a supplier. These mice were housed in polycarbonate cages, and maintained in a specific pathogen-free environment in light controlled (light on from 7:00 to 19:00) and air-conditioned rooms (temperature: 24±1°C, humidity :50 ±10%). They had free access except for the time period of electric field exposure. The animals used in this study were cared for and used under the Guiding Principles for the Care and Use of Research Animals promulgated by the Obihiro University of Agriculture of Veterinary Medicine.

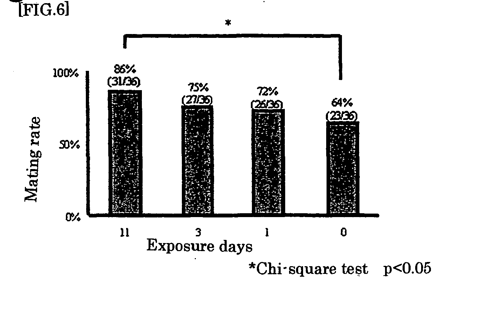

Electric field exposure system used in the present Examples is shown in Fig. 4. The electric field exposure system is composed of three major parts, namely, a high voltage unit (Healthtron, a maximum output voltage: 9000 V; Hakuju Institute for Health Science Company, Co., Ltd., Tokyo, Japan), a constant voltage unit (Tokyo Seiden Co., Ltd., Tokyo, Japan) and electric field exposure cages. As shown in Fig. 5, the exposure cage was composed of a cylindrical plastic cage (diameter 200 mm, height 200 mm) and two electrodes made of stainless steel (400×400 mm) placed over and under the cylindrical cage. Slits are provided with intervals of 5 mm in the cylindrical cage (length: 100 mm, width: 5 mm). These slits prevent such smudges as feces, saliva, and the like which disturb stable electric field formation. In order to establish the electric field (50 Hz, 45 kV/m) in the cage, a stable AC current (50 Hz, 9000 V) was applied to the upper electrode.

### (Experimental procedures)

Male mice (n=30) were placed in electric field cage and were exposed to 50 Hz, 45 kV/m electric field for 30 minutes per day for 11 days. After the final electric field exposure, the male mice were placed in a cage with a superovulated female mice (n=30) and let them mate. Superovulation was induced by intraperitoneal injections of 5IU equine chorionic gonadotrophin (eCG; Serotrophin, the Teikoku Hormone Mfg. Co., Tokyo, Japan) 2 days before the copulation and 5 I U human chorionic gonadotrophin (hCG; Puberogen, Sankyo Co., Tokyo, Japan) right before the copulation. As a control, male mice were placed in the electric field cage for 30 minutes per day for 11 days without the electric field exposure and subsequently mated with the superovulated female mice (n=30). Establishment of mating was determined by detection of a vaginal plug the morning after mating overnight.

### (Statistical Analysis)

Data presented in these Examples were analyzed statistically by chi-square test. In all statistical tests, the difference was considered significant when P was <0.05.

### (Experimental results)

In the absence of electric field exposure, the mating rates of C57BL/6J male mice with superovulated female mice were significantly lower than those of ICR male mice with superovulated female mice. When ICR and C57 BL/6J males were mated with C57 BL/6J superovulated female mice, all of the C57BL/6J female mice mated with the ICR male mice had a vaginal plug while approximately half (51%) of the female mice mated with the C57BL/6J male mice exhibited a vaginal plug. These results clearly indicate that the cause of the poor mating rates in C57BL/6J mice depends on male generative performance rather than on the female.

The effect of electric field exposure on C57BL/6J male mice mating rates with the superovulated female mice was remarkable. As shown in table 6, when male mice were exposed to 50 Hz electric field for 11 days before the mating and subsequently placed in a cage with superovulated female mice, the rate of successful mating was 90 %. While 63 % of the superovulated female mice placed with control (no electric field exposure) C57 BL/6J male mice exhibited the vaginal plug. This difference was statistically significant (P<0.05).

**Table 6**

| | No. of vaginal plug/No. of copulation (copulation rate %) |
|---|---|
| Electric field exposure groups | 27/30 (90)* |
| No electric field exposure groups | 19/30 (63) |

| | |
|---|---|
| * Significant to no electric field exposure group (P <0.05, chi-square test). | |

The male mice used in this study did not show any clinical symptoms or abnormal behavior during and after electric field exposure. The fertilization rates in the oocytes observed from the vaginal plug-positive females were not statistically different between the experimental groups (electric field exposure or no electric field exposure) (P>0.05).

To identify the essential time periods of electric field exposure to improve mating rates, C57BL/6J male mice were exposed to a 50 Hz, 45 kV/m electric field for 30 minutes per day for 1,3 and 11 days and subsequently mated with superovulated C57BL/6J female mice. According to Fig. 6, when C57BL/6J male mice were exposed to the electric field for 11 days, the mating rates with the superovulated C57BL/6J female mice were significantly improved (P<0.05), a finding similar to the results shown in table 6. However, exposure of C57BL/6J male mice to the electric field for 1 or 3 days did not significantly increase the mating rates with the C57BL/6J superovulated female mice as compared with unexposed control. These results suggest that a cumulative effect of electric exposure is required for improvement of the mating rates in C57BL/6J female mice mated with superovulated female mice.

The reason for the lower mating rate seen in C57BL/6J male mice placed with superovulated C57BL/6J female mice, but not with naturally ovulated female, is still unclear. However, this lower successful rate is one of the major difficulties in collection of a sufficiency of oocytes or embryos for transgenesis as well as research advances in embryology and developmental biology. In the present invention, it was clearly showed that the exposure of C57BL/6J male mice to electric field improved the mating rates with the superovulated female mice (table 6 and Fig.6).

It has been suggested that exposure to electric field may affect endocrine and immune systems as well as cell signaling. Since various medical instruments which harness electric field are in wide use, it has also been suggested that percutaneous electric nerve stimulation might be beneficial to patients with chronic low back pain and transcutaneous electric nerve stimulation supporting the repair of soft tissues. Furthermore, it has been reported that a mechanical vibration (5-50 Hz) to a skin activates cortex neurons corresponding to the site of stimulation. More recently, the ameliorative effects of electric field therapy on a variety of types of pain, such as headache, stiff shoulders and stomachache, have been reported in over a thousand clinical cases. However, there is as yet no report on the effect of electric field exposure on generative function in either pre-clinical or clinical studies, except for certain toxicological studies. As shown in table 6 and Fig.6, however, the exposure of C57BL/6J male mice to electric field clearly improved their sub-fertility in mating with the superovulated female mice, with a cumulative effect of the electric field exposure apparently requisite. The results in the present study appear to indicate that electric field exposure might have potential in improving male sexual function which commonly comes with aging and chronic pathologies.

Further studies must be pursued in order to elucidate the mechanism of improvement in generative function observed in C57BL/6J male mice under electric field exposure. Studies on different species are also important in finding out whether electric field exposure is effective among whole species or only among mice under specific conditions. If it is effective among whole species, there is potential for application of the electric field exposure to human beings.

As heretofore mentioned, in electric field exposure groups, compared with comparative groups, establishment of mating which is statistically significant was observed. Compared with C57BL/6J male mice with low mating rate, male mice in electric field exposure groups showed increased copulation behavior and rate of fertilized eggs by the electric field exposure. Therefore, it can be said that methods for treating and improving sexual dysfunction of the present invention is effective.

### Industrial applicability

In the present invention, sexual function of male animals can be controlled by a nonpharmacological method by electric field exposure, sexual dysfunction can be treated or improved, and sexual behavior of animals can be controlled. Therefore, sexual dysfunction not only of male human beings, but also of male animals such as horses and mice can be treated or improved. Further, the breeding of said animals can be controlled for both male and female animals.

## Claims

1. A nonpharmacological generative function control method of male animals, wherein electric field is exposed to whole or a part of said male animals with electrodes in a non-contact state with said male animals.

2. The nonpharmacological generative function control method of male animals as set forth in claim 1, wherein said electric field is a low frequency alternating current electric field.

3. The nonpharmacological generative function control method of male animals as set forth in claim 1, wherein said electric field is exposed to at least generative organs of said male animals.

4. The nonpharmacological generative function control method of male animals as set forth in claim 1, wherein said electric field is exposed only for a certain time period in a day, which is repeated everyday.

5. The non pharmacological generative function control method of male animals as set forth in claim 1, wherein the control of said sexual function includes treatment or improvement of erectile dysfunction, ejaculation dysfunction, decrease in libido or lack in orgasm.

6. A method of controlling sexual behavior of animals by using the generative function control method set forth in claim 1.

7. A generative function control apparatus for treating or improving sexual dysfunction of male animals, wherein said generative function control apparatus is provided with a pair of electrodes with electric field exposable to a whole of a part of said male animals and a power supply which applies voltage to said pair of electrodes.

8. The generative function control apparatus as set forth in claim 7, wherein said apparatus is provided with a pair of electrodes arranged with a prescribed interval so that said male animals or a part thereof might be placed and a power supply for generating electric field between said pair of electrodes by applying prescribed voltage to said pair of electrodes.

9. The generative function control apparatus as set forth in claim 7, wherein said electric field is a low frequency AC current electric field.

10. The generative function control apparatus as set forth in claim 7, wherein said apparatus is provided with a power supply controller in which at least one of applied voltage, frequency, and exposure time can be controlled.

11. The generative function control apparatus as set forth in claim 7, wherein said electric field is at least exposable to generative organs of said male animals.

12. The generative function control apparatus as set forth in claim 7, wherein said sexual dysfunction includes erectile dysfunction, ejaculation function, decrease in libido, or lack in orgasm.

13. The generative function control apparatus as set forth in claim 7, wherein said pair of electrodes is arranged in a non-contact state with said male animals.

14. The generative function control apparatus as set forth in claim 7, wherein said male animals are human beings.

15. The generative function control apparatus as set forth in claim 7, wherein said male animals are mammals.

16. The generative function control apparatus as set forth in claim 15, wherein said mammals are horses.

17. The generative function control apparatus as set forth in claim 15, wherein said mammals are mice.

18. The generative function control apparatus as set forth in claim 7, wherein a placing table on which a whole or a part of said male animals can be placed in a stable state is further provided.

19. The generative function control apparatus as set forth in claim 18, wherein said male animals are human beings and said placing table is provided with a back rest portion, a seat plate portion, and a foot rest portion on which human beings can sit.

20. The generative function control apparatus as set forth in claim 19, wherein one of said pair of electrodes is arranged upward of a head portion of said human beings by an electrode supporting portion which extends from the back rest portion of said chair and that the other of said pair of electrodes is arranged on said foot rest portion.
